# EUROPEAN PATENT SPECIFICATION

(11) **EP 3 184 146 B1**
(45) Date of publication and mention of the grant of the patent: **27.03.2019**
(21) Application number: 16205814.3
(22) Date of filing: 21.12.2016
(51) Int. Cl.: A61N 5/06, A61F 2/90, A61F 2/82, A61M 25/01, A61B 17/16, A61B 90/10

(54) **EFFICIENT DELIVERY OF PHOTOTHERAPY USING AN OPTICAL LIGHT FIBER**
EFFIZIENTE BEREITSTELLUNG VON PHOTOTHERAPIE MIT OPTISCHER LICHTFASER
DISTRIBUTION EFFICACE DE PHOTOTHÉRAPIE AU MOYEN D'UNE FIBRE OPTIQUE

(30) Priority: 22.12.2015 US 201514978321
(43) Date of publication of application: 28.06.2017
(73) Proprietor: DePuy Synthes Products, LLC, Raynham, MA 02767 (US)
(72) Inventor: PFLEIDERER, Martin, Raynham, MA 02767 (US); MARGAIRAZ, Philippe, Raynham, MA 02767 (US); LOVISA, Blaise, Raynham, MA 02767 (US); TARDY, Yanik S., Raynham, MA 02767 (US)
(74) Representative: Carpmaels & Ransford LLP

(56) References cited:
- WO-A1-2013/032984
- WO-A1-2013/114376
- US-A1- 2010 249 912
- US-A1- 2012 253 449

## Description

### BACKGROUND OF THE INVENTION

### Field of the Invention

The present invention relates to systems and devices for efficient delivery of light as a method of treatment to a target site in the human body. In particular, the present invention is directed to a stent or balloon for fixation of an optical light fiber used for delivering light therapy to a target site in the body. Furthermore, the invention is directed to a light conduit for delivery of the light through bone matter.

### Description of Related Art

Light therapy, also known as phototherapy, has been a recognized treatment of many diseases and disorders of the human body. As one illustrative example, phototherapy has been used in the treatment of diseased cervical tissue. Heretofore, most conventional methods for the delivery of light therapy to a target site within the human body have been invasive, i.e., realized by advancing an optical fiber through the tissue of the body. Madsen et al., "Development of a Novel Indwelling Balloon Applicator for Optimizing Light Delivery in Photodynamic Therapy," Lasers in Surgery and Medicine, Vol. 29: pp. 406-412 (2001) describes intracranial phototherapy with an implanted catheter and balloon diffuser which, during treatment, is accessed by percutaneous fiber optics. Such methodology imposes many drawbacks such as: (i) damage to the tissue, when the optical fiber is advance through the body; (ii) having to open dura mater; (iii) excessive heat or light, in particular, proximate the light source; (iv) having to provide either an internal power source (e.g., battery) within the implanted device or an external power source connected by electrical leads to the implantable device.

Other non-invasive methods of phototherapy illuminate target tissues from outside the body, thereby limiting their application to target sites immediately beneath the skin.

US 2012/0253449 A1 describes a stent for supporting a sidewall of a lumen which contains a first fluid. The stent comprises a stent body, a film and a photosensitizing layer. The stent body is disposed inside the lumen. The film is adapted to be penetrated by a light and covers the stent body. The photosensitizing layer is disposed on an outer surface of the film for receiving the light to generate a first object, and the film separates the photosensitizing layer and the first fluid.

US 2010/0249912 A1 describes an intraluminal device with controlled biodegradation.

WO 2013/114376 A1 describes configuring optical fibers to emit radiation by bending.

WO 2013/032984 A1 describes systems and methods for facilitating optical processes in a biological tissue.

It is therefore an object of the present invention to develop a system and method for delivery of phototherapy (e.g., light therapy of any wavelength) through a body cavity (e.g., blood vessel) or across bone structures (e.g., cortical bone plates of the skull) for the targeted treatment of diseases and/or chronic pain.

### Summary of the Invention

The present invention relates to an apparatus for efficient delivery of light to a target site in a human body, as defined in the claims.

The invention is defined in the claims, other embodiments being merely exemplary.

### Brief Description of the Drawing

The foregoing and other features of the present invention will be more readily apparent from the following detailed description and drawings of illustrative of the invention wherein like reference numbers refer to similar elements throughout the several views and in which:
Figures 1A-1C are exemplary cross-sectional views of different locations of an energy supply and light source illustrating the passage of light emitted from the light source through bone matter;
Figure 2 is a side view of an exemplary embodiment of an optical fiber substantially centered within a lumen of a stent having a cylindrical configuration except for its tapered distal and proximal ends, wherein only the distal end of the stent is secured to the optical fiber while the free end of the stent is permitted to longitudinally slide axially along the optical fiber;
Figure 3A is a side view of an exemplary cylindrical shape stent with an optical fiber secured substantially centered in its lumen by either wires or threads;
Figure 3B is an end view of the cylindrical shape stent of Figure 3A wherein the optical fiber is secured to the stent via substantially straight threads;
Figure 3C is an end view of the cylindrical shape stent of Figure 3A wherein the optical fiber is secured to the stent via non-linear wires;
Figure 4A is a side view illustration of how the radially outward supporting members or struts in the stent depicted in Figure 3A causes certain shadowing of the diffuser inside the stent;
Figure 4B is a side view of an exemplary embodiment in which an optical fiber is passed through and substantially centered within lumen of two respective cylindrical shape stents (each of which is as illustrated in Figure 3A) while the light is emitted from the optical fiber at an unobstructed location between the two stents;
Figure 5A is a side view of an exemplary cylindrical shape stent with an optical fiber extending longitudinally therethrough and being maintained substantially centered in its lumen wherein the optical fiber has a bendable frontal diffuser;
Figure 5B is a side view of an exemplary cylindrical shape stent with an optical fiber extending longitudinally therethrough being maintained substantially centered in its lumen wherein the light exiting from the optical fiber is reflected by an angled mirror to a desired target site;
Figure 6 is a side view of an exemplary cylindrical shape stent in which one half of the stent is covered with an opaque material while the other half is covered by a transparent material;
Figure 7A is a side view of an exemplary delivery catheter having a longitudinal slit defined therein for enlarging its diameter;
Figure 7B is a side view of an exemplary delivery catheter having a spiral slit defined therein for enlarging its diameter;
Figure 8A is a side view of an exemplary stent having multiple light fibers on its outer surface or woven into the mesh;
Figure 8B is an end view of the stent in Figure 8A; and
Figure 9 is a perspective view of the optical fiber suspended between the 3rd and 4th ventricle of the human body.

### Detailed Description of the Invention

The terms "proximal"/"proximally" and "distal"/"distally" refer to a direction closer to or away from, respectively, an operator (e.g., surgeon, physician, nurse, technician, etc.) who would insert the medical device into the patient, with the tip-end (i.e., distal end or leading end) of the device inserted inside a patient's body. Thus, for example, a "proximal direction" would refer to the direction towards the operator, whereas "distal direction" would refer to the direction away from the operator towards the leading or tip-end of the medical device.

When delivering phototherapy to structures deep within the human body (e.g., within the brain) a light emitting optical fiber or other light source may be placed inside an artery, vein or blood vessel or other target site at least temporarily, if not for an extended period of time. Typically, the optical fiber is advanced or steered to a target site inside a blood vessel using a guide wire and delivery catheter. Specifically, delivery of the stent with the fiber optic light disposed therein to a target site in the human body is accomplished by inserting a steerable guide wire to the desired target site in the human body. Advancement of the guide wire to its intended target site may be aided by an X-ray or other imaging device. Once the guide wire is properly positioned at its intended target site, a delivery catheter is then inserted over the guide wire. The guide wire is then retracted from the human body by pulling in a proximal direction. While in its retracted/compressed state, a stent with the fiber optics disposed in its lumen is then advance through the delivery catheter to a target site in the body. Depending on whether the stent is self-expanding or not, after emerging from the distal end of the delivery catheter the stent is deployed from its retracted/compressed state to an expanded state. In the case of a self-expanding stent, upon exiting from the distal end of the delivery catheter at the target site, the stent automatically reverts to its expanded state. Otherwise, if a mechanical expanding stent is used, after it has passed through the distal end of the delivery catheter, a balloon or other mechanical device may be deployed into the lumen of the stent and inflated to expand the stent. Either type of stent is contemplated and within the intended scope of the present invention. When using a self-expanding stent, friction is generated during advancement of the stent through the delivery catheter. Once the stent has been expanded (either self-expanding or via the deployment of an inflation device) the delivery catheter is retracted from the human body by pulling in a proximal direction while leaving in place the expanded stent properly positioned at the target site in the body.

During an open intracranial operation, the steer guide wire described in the preceding paragraph may be eliminated altogether, when the stent and fiber optics disposed in the lumen thereof is inserted through one or more of the ventricles (i.e., four connected fluid-filled cavities in the center of the brain). In this alternative method of delivery of the assembled stent and fiber optics, a delivery catheter with a stylet is introduced into the ventricles during the open intracranial operation. Once the distal end of the delivery catheter is positioned at the target site in the body, the stylet is retracted or withdrawn from the body leaving in place the delivery catheter. The assembled stent and fiber optics is then inserted via the catheter to the target site in the body. Once the stent has been expanded at the desired target site in the body, the delivery catheter is retracted or withdrawn from the body by pulling in a proximal direction leaving in place the properly positioned fiber optics.

Once properly advanced using the delivery catheter to the desired location in a cavity of the body (e.g., cavities formed by the ventricular system or the central nervous system or by the vascular system such as blood vessels or the sphenoid sinus (S.S.)), it is desirable to secure or maintain in place the optical light fiber inside of the cavity while simultaneously insuring that the tip of the light emitting optical fiber stays clear of tissue that may otherwise become damaged by heat or relatively high local irradiance density. To achieve this end, when illuminating with light in an anatomical cavity of the human body it is beneficial to maintain the light source (e.g., optical fiber) in a predetermined position (e.g., substantially centered within the cavity). Such intraluminal fixation may be accomplished by anchoring or securing the light source or diffuser within a separate physical structure (e.g., a stent, cage or balloon) implanted within the same cavity of the human body as the light source.

In the case of the stent, the separate physical structure is preferably in the form of a mesh structure or cage. The stent may be helical/spiral, cylindrical, cone shape or some other geometric configuration. Furthermore, the stent may either be self-expanding (i.e., automatically expands upon removal of a mechanical force maintaining it in a radially retracted/restricted/compressed state) or non-self-expanding (i.e., requiring a separate mechanical device to insure that the stent transitions from a retracted/restricted/compressed state to an expanded state). While in an expanded state, the outer surface of the stent physically contacts the inner surface of the cavity (e.g., blood vessel) in which it is disposed thereby securing the stent in place. Instead of a stent, a balloon may alternatively be used to substantially center the light diffuser/source (e.g., fiber optics) as well as to completely fill the volume of the cavity (e.g., S.S.) to prevent build up of fluid and possible infection.

The fiber optics may be maintained in a predetermined location, preferably substantially centered, in the lumen of the stent by the design or configuration of the stent itself. One way to accomplish this result is to secure, attach or affix at least a portion, for example, one end or extremity, of the stent to the light fiber.

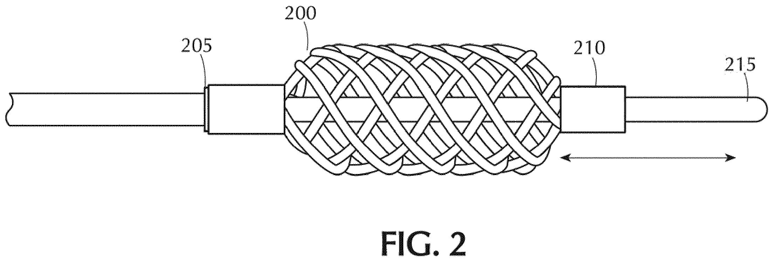

Referring to an exemplary embodiment depicted in Figure 2 both the proximal and distal ends 210, 205, respectively, of stent 200 may be narrowly tapered in opposing directions with the center region of the stent having a substantially uniform diameter. The narrowly tapered ends in both directions insure substantial centering of the stent at both ends, rather than only at one end. Centering at both ends provides greater stability with the optical fiber maintained substantially concentric relative to the stent over a predetermined longitudinal length, rather than only at a single point. Such configuration is particularly well suited for a lateral diffuser. Despite the tapering at both ends of the stent, only one end (e.g., distal end 205) of the stent 200 in Figure 2 is attached, secured or affixed to the optical fiber 215. The opposite end (e.g., proximal end 210) of the stent 200, despite being narrowly tapered in a proximal direction, is not attached, secured or affixed to the optical fiber 215. Instead, during deployment the end (e.g., proximal end 210) of the sent 400 that is not attached or affixed to the optical fiber is allowed to freely slide in a longitudinal direction axially along the optical fiber 215 (as denoted by the bidirectional arrow). That portion of the stent in Figure 2 which is attached, secured or affixed to the light fiber is at least partially, if not completely, prohibited from fully expanding. As an alternative embodiment, one end (e.g., distal end 205) of the stent 200 is attached, secured or affixed to the optical fiber 215 itself, while its opposite end (e.g., proximal end 210)) is attached, secured or affixed to a separate structure (e.g., a ring or other component encircling at least a portion of the optical fiber) that is allowed to slide longitudinally along the optical fiber to accommodate deployment of the stent.

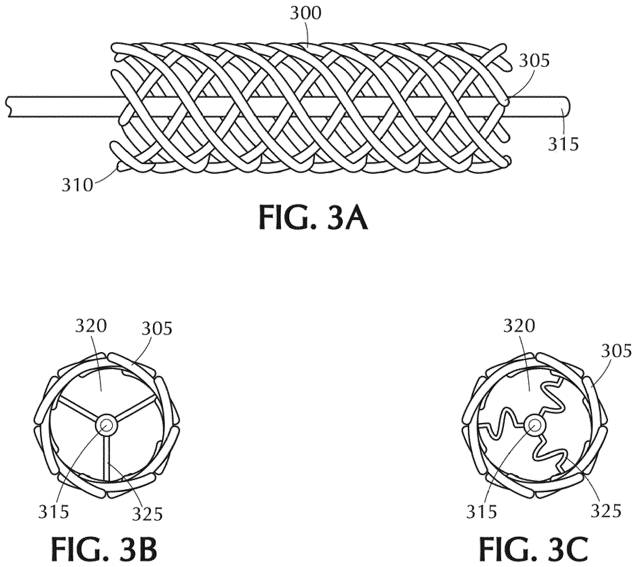

Figures 3A-3C illustrate yet another configuration of the stent to insure that the optical fiber disposed in the lumen thereof is substantially centered therein. Rather than have at least one narrowly tapered end as in the embodiments shown in Figures 2A & 2B, the stent 300 in Figure 3A is a cylinder shape tube of substantially uniform diameter from its proximal end 305 to its opposite distal end 310 (without any tapering at either its distal or proximal end). A light fiber 315 is suspended so as to be substantially centered in the lumen 320 by one or more radially outward supporting members or struts 325 such as wires (e.g., biocompatible metal)(Figure 3C) or supporting threads (e.g., biocompatible polymer)(Figure 3B). The supporting members 325 may be either linear (Figure 3B) or non-linear (Figure 3C). Since the only physical obstruction in the lumen 320 of the stent 300 in Figure 3A is the supporting members 325, obstruction of blood or CSF flow through the lumen of the stent is reduced or minimized relative to that of the stent configuration in Figure 2. By way of example, Figures 3A-3C depict three radially outward supporting members or struts 325. However, any number of one or more radially outward supporting members or struts is contemplated and within the scope of the invention.

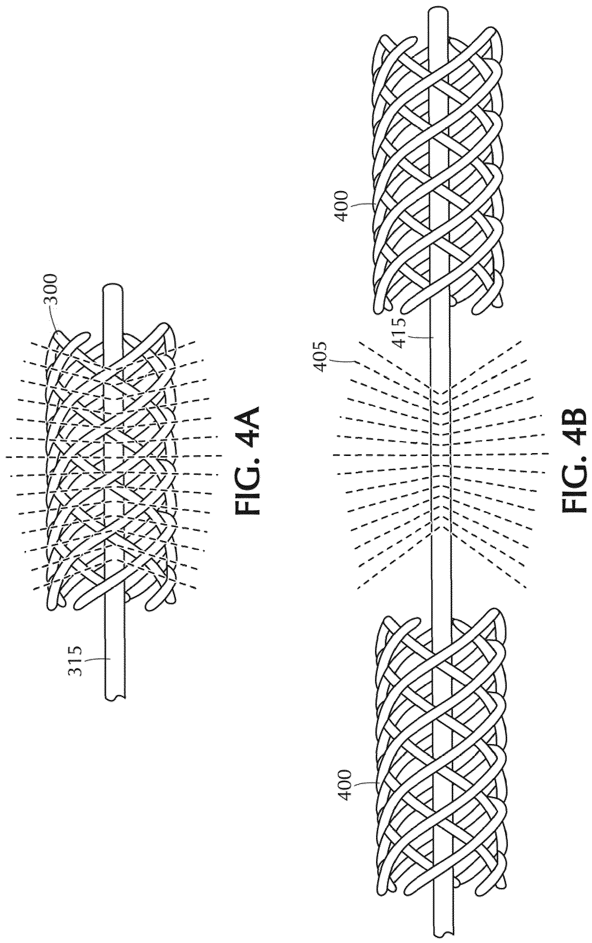

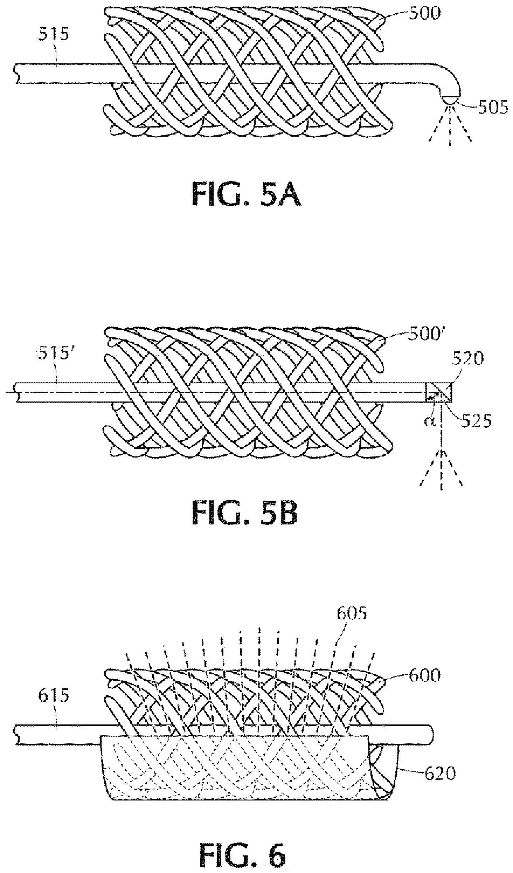

Irrespective of the configuration of the stent, the radially outward supporting members or struts 325 forming the mesh stent 300 are, preferably, least dense in a transition region in which the diameter of the stent increases located at one of its proximal or distal ends in order to minimize obstruction and optimize blood or CSF flow through the lumen. In the embodiments shown in Figures 3A-3C, the radially outward supporting members or struts 325 that form the mesh stent 300 may disadvantageously obstruct the propagation of light into the surrounding tissue or cause shadowing if the emitted light from the optical fiber 315 is radially diffused from within the lumen 320 of the stent 300, as illustrated in Figure 4A. Such undesirable obstruction or shadowing may be avoided by using two stents 400 with the radially diffused 405 emitted light from the optical fiber 415 disposed between them, as illustrated in exemplary embodiment shown in Figure 4B. Each stent 400 may be configured in accordance with that described in detail with respect to Figure 3A. Another approach to avoid possible shadowing of the struts of the stent 500 shown in Figure 5A is to emit the light from the optical fiber 515 directionally from one of its ends (e.g., using a frontal diffuser 505), rather than radially diffused between its ends (as shown in Figure 4B). After proper placement at the target site, the angle and direction of the radiated light emitted by the optical fiber may be selected, as desired, by bending the end of the frontal diffuser 505. An alternative embodiment is illustrated in Figure 5B. Instead of a frontal diffuser as illustrated in Figure 5A, a mirror 520 may be positioned at the distal end of the optical fiber 515', whereby the light exiting from the optical fiber 515' is reflected by the mirror 520 and is transmitted through a transparent surface 525. In the preferred embodiment depicted in Figure 5B, mirror 520 is oriented at an angle α of approximately 45°. However, any desired angle α is contemplated and within the intended scope of the invention to focus or direct the radiated light onto the target site.

To further concentrate, focus or amplify reflection of the emitted light from the optical fiber in a preferred direction, the stent 600 may be partially covered by an opaque material 620 (e.g., metal foil) that reduces, obstructs, prohibits or prevents radially diffused light 605 emitted from the optical fiber 615 from passing through the opaque material, as shown in exemplary Figure 6. Note that the refractive properties of the metal foil serves the dual purpose of shielding a portion of the surrounding tissue from illumination while simultaneously amplifying illumination of the light 605 in the preferred direction.

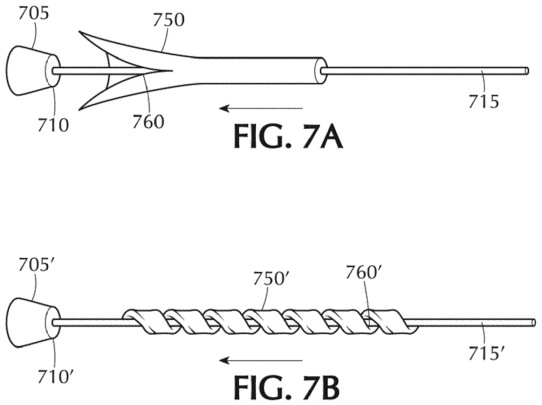

In any of the embodiments described herein, a connecting member, such as a plug or light port connected to the light source, disposed at the proximal end of the light fiber may have an outer diameter larger than the inner diameter of the delivery catheter through which it is advanced. As a result, withdrawal, retrieval or removal of the delivery catheter following implantation of the optical fiber at the target site may be obstructed by such connecting member having an enlarged diameter. To overcome this problem, the connecting member may be designed or configured to cut through, split, expand, open, unravel or separate the delivery catheter as it is removed from the body by pulling in a proximal direction. Figure 7A shows one exemplary configuration in which the connecting member 705 is designed or configured to cut through, open or separate a slit 760 extending in a substantially longitudinal direction of the delivery catheter 750 from its proximal end completely through to its opposite distal end as the delivery catheter is pulled in a proximal direction during retrieval from the human body. By way of illustrative example shown in Figure 7A, disposed on the distal end of the connecting member or light port 705 associated with the light source is a projection or nub 710 substantially aligned so as to physically interfere with a longitudinally defined slit or opening 760 in the delivery catheter 750. As the delivery catheter 750 is pulled in a proximal direction the projection or nub 710 is introduced into the longitudinally defined slit 760 causing a separation therein starting from its proximal end completely through to its opposite distal end. The longitudinal slit, separation or opening 760 created in the delivery catheter 750 enlarges the inner diameter of the delivery catheter allowing it to pass over the connecting member 705 which remains implanted in the body along with the light fiber at the desired location to illuminate the target site. As an alternative, Figure 7B depicts a design of the delivery catheter 750' that cuts, splits, expands, separates, opens, unravels or peels along a spiral or helical configured slit 760'. Once again, as the delivery catheter 750' is pulled in a proximal direction from the human body, the nub or projection 710' penetrates the spiral slit 760' enlarging the inner diameter of the delivery catheter 750' to exceed that of the connecting member 705' so that the delivery catheter 750' may be recovered from the human body leaving in place the fiber optics and connecting member 705'.

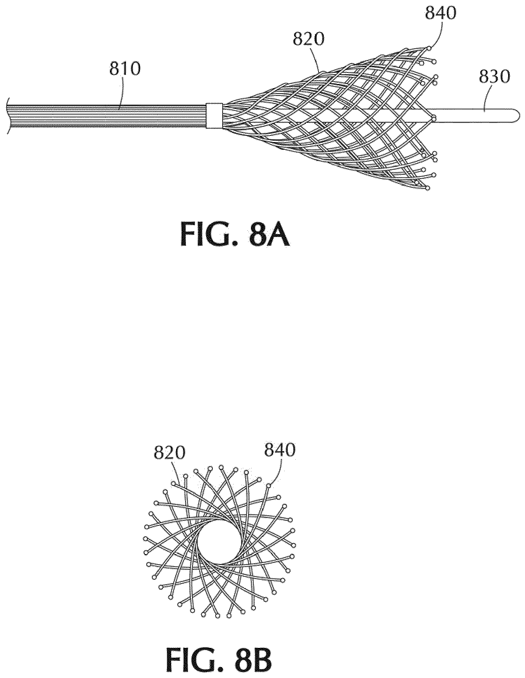

Multiple light fibers 840 may be disposed on the outer and/or inner surface of the stent 820, as illustrated in Figures 8A & 8B. Specifically, the multiple light fibers 840 may be integrated into the covering disposed on the outer surface of the stent, as shown in Figure 8B. In addition, the covering and multiple light fibers may be coated from the inside with a reflective metal to further direct the illuminated light externally radially outward, rather than internally radially inward. Once again, as described above with respect to the other embodiments, the stent 820 may be advanced though a cavity (e.g., blood vessel) using a delivery catheter 810 and guide wire 830.

The stent in accordance with any of the embodiments described herein may be covered by a material for dispensing a drug once the stent has been implanted in the body. For example, the stent may be covered with a polymer material (e.g., polyurethane) impregnated with a drug (e.g., paclitaxel). Once the stent has been implanted, the drug (e.g., paclitaxel) may be slowly released into the body around the stent.

In all the embodiments described herein the optical fiber or light fiber may be replaced by an electrical cable and one or more light emitting diodes (LEDs).

As an alternative to the previously described stent, a balloon also may be used as a physical structure to maintain the light source in a substantially centered position. Due to its closed structure, the balloon serves the dual function of insuring the complete filling of the volume of the cavity to be irradiated. In the case of illuminating the S.S. with light, the complete filling of the volume realized by using a balloon serves an additional benefit of reducing build up of fluid and thus possible infection. Anchoring of the light source within the separate implanted physical structure (e.g., stent or balloon) more evenly distributes the light radially 360 degrees thereby minimizing damage to the adjacent tissue due to excessive light energy density and/or heat.

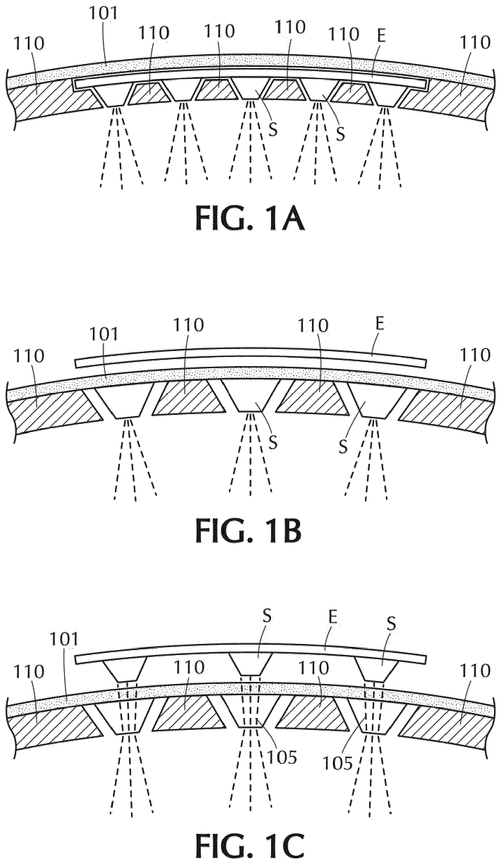

Rather than passing the fiber optics, light fiber or other light source itself within a vessel or other cavity of the body to a target site, a light conduit or diffuser may be used as a conduit for transmitting light emitted from the light source through bone without any portion of the light source itself being inserted or disposed in the bone. In this delivery method, phototherapy from the light source disposed on one side of bone matter may be delivered to a target site disposed on the opposite side of the bone matter using a light conductor or diffuser 105. In an exemplary embodiment depicted in Figure 1C, the energy supply (E) and light source (S) both are disposed exteriorly/outside of the body 110 (i.e., on top of the scalp 101). With this configuration, the light source (S) is arranged on one side of the skull 110, while the target site (e.g., S.N.) is disposed on an opposite site of the skull 110. Light conductors or diffusers 105, for example, transparent screws, dowels or plugs, that fully penetrate the bone matter 110 (e.g., skull) serves as a conduit to transmit the emitted light therethrough. In essence, one or more light conductors or diffusers 105 act as a plug inside a corresponding hole of the bone. Such light conductor or diffuser 105 is said to fully penetrate the bone matter 110 when one end of the light conductor or diffuser 105 projects/exposed from one side of the bone matter, while its opposite end projects/exposed from an opposite side of the bone matter. The light conductor or diffuser may be made of any material that is transparent, able to transmit light and biocompatible, such as, glass, acrylic glass, PMMA, PVC.

One or more holes is drilled in the bone matter 110 while preserving (i.e., without penetrating) the dura mater 115. Each hole is then fitted with at least one light conductor or diffuser 105. The individual light conductors or diffusers 105 may be independent of each other. Otherwise, one or more light conductors or diffusers 105 may be connected via a plate 120. The plate is made of a material that is transparent, able to transmit light and biocompatible, such as, glass, acrylic glass, PMMA or PVC. Materials used for the light conductors or diffusers 105 and plate 120 may, but need not necessarily be, the same. By way of illustrative example, one or more light conductors or diffusers 105 may be implanted in the bone matter to transmit one or more light beams from a light source (e.g., optical fiber) to a target site (e.g., toward the Substantia Nigra (S.N.)). This system and method of phototherapy to the brain is particularly beneficial in that it provides a more even distribution of irradiated light and heat energy from multiple directions to the S.N.

Hence, the light conductor or diffuser 105 transmits the light emitted by the light source (S) disposed on one side of the bone matter 110 to the target site disposed on an opposite side of the bone matter 110. Light of any desired wavelength range generated by the light source (S) and powered by an energy supply (E) illuminates a first end of the light conduit or diffuser 105 that acts as a light conduit through the bone emitting the light from its opposite end thereby illuminating the target site. If the transparent plugs 105 in Fig 1C were eliminated a portion of the light emitted by the light source (S) would disadvantageously be absorbed by the bone 110.

Not only is the light conduit or diffuser (e.g., bone screw or bone plug) able to transmit through bone matter light emitted from a light source, it may also serve the dual purpose of receiving light reflected off the target site (e.g., tissue or bone). From the received light reflected off the target site (e.g., tissue or bone), the irradiance inside the tissue or the thickness of the bone may be estimated. Based on this data, the level of light emissions from the light source may be adapted in a feedback loop to optimize phototherapy treatment.

Figure 1C shows a configuration in which the energy supply (E) and light source (S) both are disposed exteriorly/outside of the scalp 101 and skull 110 to irradiate the S.N. However, the energy supply (E) and/or light source (S) may be disposed either externally (e.g., Radio Frequency) of the body or implanted in the body. If the light source (S) fully penetrates the bone matter 110 then there is no need for a light diffuser or conduit 105 as described in Figure 1C. Figures 1A & 1B depict different configurations in which the light source (S) fully penetrates the bone matter 110. In particular, Figure 1A is an exemplary embodiment in which the energy supply (E) and light source (S) are subcutaneously placed interiorly/inside of the bone 110 (e.g., the skull). Because the light source (S) fully penetrates the bone 110, the bone 110 does not present a barrier to the light. An alternative embodiment as illustrated in Figure 1B depicts the energy supply (E) exterior/outside of the body 110 (i.e., on top of the scalp) while the light source (S) is disposed interiorly/inside the bone 110. Similar to that in Figure 1A, the light source (S) in Figure 1B once again fully penetrates the bone 110 and therefore the bone 110 does not present a barrier to the light.

The present inventive system is particularly well suit to phototherapy of the S.S. The delivery of phototherapy to the S.S may be accomplished by forwarding fiber optics from the femoral artery to the carotid artery (at the level of the S.N.) using conventional guide wire technology under X-ray or other imaging technology. Since the fiber optics is advanced via an artery, there is no damage to tissue during advancement. In this method of delivery of the fiber optics once implanted to a target site within the body via the
artery, the guide wire may be removed leaving in place the implanted fiber optics for phototherapy treatment of a disease over an extended period of time. This may be particular well suited in the treatment or reduction of chronic pain using phototherapy. As an alternative to delivery through an artery, the fiber optics may be advance through a puncture made in the spinal cord.

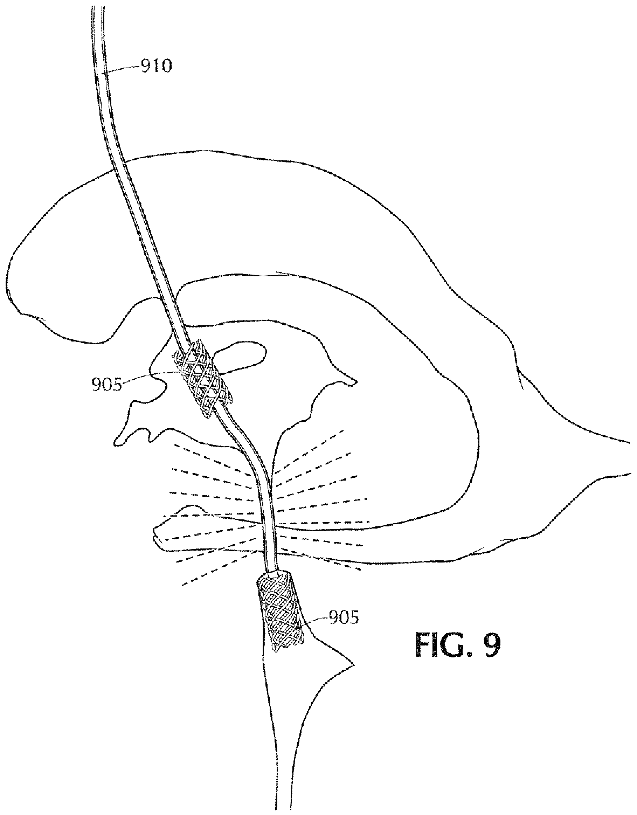

During phototherapy treatment of the Substantia Nigra (S.N.) of the brain stem for the treatment of neurological disease (e.g., Parkinson's disease) and/or chronic pain one of the inventive systems and procedures, as described in detail above, may be used as the method of fixation of the light fiber at a target site within the body. In a particular application, one or more fiber optics 910 may be positioned in the Aqueduct of Silvius with the preferred points of fixation of such light fibers between the 3^{rd} and 4^{th} ventricles of the brain using one or more stents 905, as depicted in Figure 9. Fixation or anchoring of the fiber optics at this location using one or more stents in accordance with one of the previously described embodiments advantageously does not interfere with the circulation of cerebral spinal fluid (CSF) through the intraventricular foramen. Furthermore, due to the relatively small diameter of the light fiber (preferably less than approximately 1mm, most preferably approximately 0.4mm), the Aqueduct of Silvius is not damaged by the light source.

Alternatively a diffuser or light conduit inside the S.S. can be more easily supplied with light if a fiber optics connects to a more readily accessible anatomical structure such as the forehead. The fiber optics between the site of light introduction into the body and the S.S. is channeled through the interior soft bone tissue (Spongiosa) of the skull bone plates. Hence, an external fiber optics light source produces light that is pushed through the bone plates to a target site within the brain using a light conductor as previously described herein. The light diffuser or light conduit not only emits light but may also receive reflected light in order to estimate the effective irradiance inside the bone or tissue. Based on the estimations, the level of light emission from the source may be adapted in a feedback loop in order to maintain a desired irradiance most suitable to achieve the intended phototherapeutic effect.

The light source may be implanted within the patient's body thereby obviating the need to transport the light from outside the body through skin and bone structures into the body. Energy supplied to the implanted light source may be effectuated electromagnetically at radio frequency or via an implanted battery.

The present inventive system for phototherapy provides many advantages such as minimally invasive access for light conducting fibers to structures deep inside the brain (e.g., via blood vessels or inside the bone). The light source may be internal/implanted with either an internal or external power supply thereby eliminating having to relay the light through the skin and/or bone structure. Moreover, because the physical structure (e.g., stent or balloon) maintains the optical fiber a predetermined distance relative to the tissue or bone, potential damage to tissues or bone closest to the light source due to high irradiance and/or heat is reduced or avoided. Lastly, externally routing, rather than internally introducing the fiber optics into the brain or soft tissue, reduces damage to delicate brain tissue.

Thus, while there have been shown, described, and pointed out fundamental novel features of the invention as applied to a preferred embodiment thereof, it will be understood that various omissions, substitutions, and changes in the form and details of the devices illustrated, and in their operation, may be made by those skilled in the art without departing from the scope of the invention. For example, it is expressly intended that all combinations of those elements and/or steps that perform substantially the same function, in substantially the same way, to achieve the same results be within the scope of the invention. Substitutions of elements from one described embodiment to another are also fully intended and contemplated. It is also to be understood that the drawings are not necessarily drawn to scale, but that they are merely conceptual in nature. It is the intention, therefore, to be limited only as indicated by the scope of the claims appended hereto.

## Claims

1. An apparatus for efficient delivery of light to a target site in a human body, the apparatus comprising:
a light source (215);
at least one stent (200) having a lumen defined longitudinally therethrough;
**characterised in that**:
the light source is an optical fiber or an electrical cable and one or more light emitting diodes disposed longitudinally within the lumen of the stent, wherein the stent has a tapered first end (205) and a tapered opposite second end (210), and wherein only the first end of the stent is secured to the optical fiber while the opposite second end of the stent is permitted to longitudinally slide axially along the light source.

2. The apparatus according to claim 1, wherein the stent has a cylindrical shape of substantially uniform diameter in a region between its first and second tapered ends.

3. An apparatus for efficient delivery of light to a target site in a human body, the apparatus comprising:
a light source (315);
at least one stent (300) having a lumen defined longitudinally therethrough;
**characterised in that**:
the light source is an optical fiber or an electrical cable and one or more light emitting diodes disposed longitudinally within the lumen of the stent, wherein the stent has a cylindrical shape of substantially uniform diameter from a first end (310) to an opposite second end (305), and the light source is suspended within the lumen of the stent by at least one radially outward supporting member (325).

4. An apparatus for efficient delivery of light to a target site in a human body, the apparatus comprising:
a light source (415);
**characterised in that**:
the apparatus includes two stents, each having a lumen defined longitudinally therethrough, and the light source is an optical fiber or an electrical cable and one or more light emitting diodes disposed longitudinally within the lumen of each stent, and wherein light is radially emitted from the light source in a region between the two stents.

5. The apparatus according to any one of claims 1 to 4, wherein the light source is an optical fiber, the apparatus further comprising a frontal diffuser (505) disposed at a distal end of the optical fiber from which light is emitted; the frontal diffuser being adjustable in at least one of direction and angle.

6. The apparatus according to any one of claims 1 to 4, wherein the light source is an optical fiber, the apparatus further comprising a mirror (520) positioned at a distal end of the optical fiber to reflect light exiting from the optical fiber before being transmitted through a transparent surface (525).

7. The apparatus according to any one of claims 1 to 3, wherein a portion of the stent is opaque (620), while its remaining portion is transparent.

8. The apparatus according to any preceding claim, wherein the light source (715) has a proximal end on which is disposed a connecting member (705) with a projection (710) extending radially outward therefrom; the apparatus further comprising a delivery catheter with a slit (760) defined longitudinally therethrough; the connecting member having an outer diameter greater than an inner diameter of the delivery catheter; the projection being receivable within the slit causing the slit to separate thereby enlarging the inner diameter of the delivery catheter allowing the connecting member to remain in position within the body as the delivery catheter is withdrawn by pulling in a proximal direction.

9. The apparatus according to any one of claims 1 to 7, wherein the light source (715') has a proximal end on which is disposed a connecting member (705') with a projection (710') extending radially outward therefrom; the apparatus further comprising a delivery catheter (750') with a spiral slit (760') defined longitudinally therethrough; the connecting member having an outer diameter greater than an inner diameter of the delivery catheter; the projection being receivable within the slit causing the slit to separate thereby enlarging the inner diameter of the delivery catheter allowing the connecting member to remain in position within the body as the delivery catheter is withdrawn by pulling in a proximal direction.

## Patentansprüche

1. Vorrichtung für die wirksame Abgabe von Licht an einen Zielort in einem menschlichen Körper, wobei die Vorrichtung umfasst:
eine Lichtquelle (215);
wenigstens einen Stent (200) mit einem in Längsrichtung hindurchgehend definierten Lumen;
**dadurch gekennzeichnet, dass**:
die Lichtquelle eine optische Faser oder ein elektrisches Kabel und eine oder mehrere lichtemittierende Dioden ist, die in Längsrichtung innerhalb des Lumens des Stents angeordnet ist, wobei der Stent ein verjüngtes erstes Ende (205) und ein verjüngtes, gegenüberliegendes zweites Ende (210) aufweist, wobei nur das erste Ende des Stents an der optischen Faser befestigt ist, während das gegenüberliegende zweite Ende des Stents in Längsrichtung axial entlang der Lichtquelle gleiten kann.

2. Vorrichtung gemäß Anspruch 1, wobei der Stent eine zylindrische Form mit einem im Wesentlichen gleichmäßigem Durchmesser in einem Bereich zwischen seinem ersten und seinem zweiten verjüngten Ende aufweist.

3. Vorrichtung für die wirksame Abgabe von Licht an einen Zielort in einem menschlichen Körper, wobei die Vorrichtung umfasst:
eine Lichtquelle (315);
wenigstens einen Stent (300) mit einem in Längsrichtung hindurchgehend definierten Lumen;
**dadurch gekennzeichnet, dass**:
die Lichtquelle eine optische Faser oder ein elektrisches Kabel und eine oder mehrere lichtemittierende Dioden ist, die in Längsrichtung innerhalb des Lumens des Stents angeordnet ist, wobei der Stent eine zylindrische Form mit einem im Wesentlichen gleichmäßigem Durchmesser von einem ersten Ende (310) zu einem gegenüberliegenden zweiten Ende (305) aufweist und die Lichtquelle innerhalb des Lumens des Stents durch wenigstens ein radial nach außen ragendes Stützelement (325) aufgehängt ist.

4. Vorrichtung für die wirksame Abgabe von Licht an einen Zielort in einem menschlichen Körper, wobei die Vorrichtung umfasst:
eine Lichtquelle (415);
**dadurch gekennzeichnet, dass**:
die Vorrichtung zwei Stents umfasst, die jeweils ein in Längsrichtung hindurchgehend definiertes Lumen aufweisen, und die Lichtquelle eine optische Faser oder ein elektrisches Kabel und eine oder mehrere lichtemittierende Dioden ist, die in Längsrichtung innerhalb des Lumens jedes Stents angeordnet ist, wobei in einem Bereich zwischen den beiden Stents Licht radial von der Lichtquelle emittiert wird.

5. Vorrichtung gemäß einem der Ansprüche 1 bis 4, wobei die Lichtquelle eine optische Faser ist, die Vorrichtung ferner einen vorderen Diffusor (505) umfasst, der an einem distalen Ende der optischen Faser angeordnet ist, aus der Licht emittiert wird; wobei der vordere Diffusor in wenigstens einem von Richtung und Winkel einstellbar ist.

6. Vorrichtung gemäß einem der Ansprüche 1 bis 4, wobei die Lichtquelle eine optische Faser ist, die Vorrichtung ferner einen Spiegel (520) umfasst, der an einem distalen Ende der optischen Faser angeordnet ist, um Licht zu reflektieren, das aus der optischen Faser austritt, bevor es durch eine transparente Oberfläche (525) durchgelassen wird.

7. Vorrichtung gemäß einem der Ansprüche 1 bis 3, wobei ein Teil des Stents undurchsichtig (620) ist, während sein restlicher Teil transparent ist.

8. Vorrichtung gemäß einem der vorstehenden Ansprüche, wobei die Lichtquelle (715) ein proximales Ende aufweist, an dem ein Verbindungselement (705) mit einem Vorsprung (710) angeordnet ist, der davon radial nach außen ragt; wobei die Vorrichtung ferner einen Abgabekatheter umfasst, der einen in Längsrichtung hindurchgehend definierten Schlitz (760) aufweist; wobei das Verbindungselement einen Außendurchmesser aufweist, der größer als der Innendurchmesser des Abgabekatheters ist; wobei der Vorsprung in dem Schlitz aufgenommen werden kann, um Trennen des Schlitzes zu bewirken und dadurch den Innendurchmesser des Abgabekatheters zu vergrößern und zu erlauben, dass das Verbindungselement an seiner Position innerhalb des Körpers bleibt, wenn der Abgabekatheter durch Ziehen in einer proximalen Richtung zurückgezogen wird.

9. Vorrichtung gemäß einem der Ansprüche 1 bis 7, wobei die Lichtquelle (715') ein proximales Ende aufweist, an dem ein Verbindungselement (705') mit einem Vorsprung (710') angeordnet ist, der davon radial nach außen ragt; wobei die Vorrichtung ferner einen Abgabekatheter (750') mit einem in Längsrichtung hindurchgehend definierten Spiralschlitz (760') aufweist; wobei das Verbindungselement einen Außendurchmesser aufweist, der größer als der Innendurchmesser des Abgabekatheters ist; wobei der Vorsprung in dem Schlitz aufgenommen werden kann, um Trennen des Schlitzes zu bewirken und dadurch den Innendurchmesser des Abgabekatheters zu vergrößern und zu erlauben, dass das Verbindungselement an seiner Position innerhalb des Körpers bleibt, wenn der Abgabekatheter durch Ziehen in einer proximalen Richtung zurückgezogen wird.

## Revendications

1. Appareil pour l'administration efficace de lumière à un site cible dans un corps humain, l'appareil comprenant :
une source de lumière (215) ;
au moins une endoprothèse (200) ayant une lumière définie longitudinalement à travers celle-ci ;
**caractérisé en ce que** :
la source de lumière est une fibre optique ou un câble électrique avec une ou plusieurs diodes électroluminescentes disposées longitudinalement à l'intérieur de la lumière de l'endoprothèse, l'endoprothèse ayant une première extrémité effilée (205) et une seconde extrémité opposée effilée (210), et seule la première extrémité de l'endoprothèse étant fixée à la fibre optique tandis que la seconde extrémité opposée de l'endoprothèse peut coulisser longitudinalement axialement le long de la source de lumière.

2. Appareil selon la revendication 1, l'endoprothèse ayant une forme cylindrique de diamètre sensiblement uniforme dans une région entre ses première et seconde extrémités effilées.

3. Appareil pour l'administration efficace de lumière à un site cible dans un corps humain, l'appareil comprenant :
une source de lumière (315) ;
au moins une endoprothèse (300) ayant une lumière définie longitudinalement à travers celle-ci ;
**caractérisé en ce que** :
la source de lumière est une fibre optique ou un câble électrique avec une ou plusieurs diodes électroluminescentes disposées longitudinalement à l'intérieur de la lumière de l'endoprothèse, l'endoprothèse a une forme cylindrique de diamètre sensiblement uniforme d'une première extrémité (310) à une seconde extrémité opposée (305), et la source de lumière est suspendue à l'intérieur de la lumière de l'endoprothèse par au moins un élément de support radialement vers l'extérieur (325).

4. Appareil pour une administration efficace de lumière à un site cible dans un corps humain, l'appareil comprenant :
une source de lumière (415) ;
**caractérisé en ce que** :
l'appareil comprend deux endoprothèses, chacune ayant une lumière définie longitudinalement à travers celle-ci, et la source de lumière est une fibre optique ou un câble électrique avec une ou plusieurs diodes électroluminescentes disposées longitudinalement à l'intérieur de la lumière de chaque endoprothèse, et la lumière étant émise radialement à partir de la source de lumière dans une région entre les deux endoprothèses.

5. Appareil selon l'une quelconque des revendications 1 à 4, la source de lumière étant une fibre optique, l'appareil comprenant en outre un diffuseur frontal (505) disposé à une extrémité distale de la fibre optique à partir de laquelle la lumière est émise ; le diffuseur frontal étant réglable dans au moins une direction et un angle.

6. Appareil selon l'une quelconque des revendications 1 à 4, la source de lumière étant une fibre optique, l'appareil comprenant en outre un miroir (520) positionné au niveau d'une extrémité distale de la fibre optique pour réfléchir la lumière sortant de la fibre optique avant d'être transmise à travers une surface transparente (525).

7. Appareil selon l'une quelconque des revendications 1 à 3, une partie de l'endoprothèse étant opaque (620), tandis que sa partie restante est transparente.

8. Appareil selon l'une quelconque des revendications précédentes, la source de lumière (715) ayant une extrémité proximale sur laquelle est disposé un élément de liaison (705) avec une saillie (710) s'étendant radialement vers l'extérieur à partir de celui-ci ; l'appareil comprenant en outre un cathéter de distribution ayant une fente (760) définie longitudinalement à travers celui-ci ;
l'élément de liaison ayant un diamètre externe supérieur à un diamètre interne du cathéter de distribution ;
la saillie pouvant être reçue à l'intérieur de la fente provoquant la séparation de la fente, ce qui permet d'agrandir le diamètre interne du cathéter de distribution permettant à l'élément de liaison de rester en position à l'intérieur du corps lorsque le cathéter de distribution est retiré par traction dans une direction proximale.

9. Appareil selon l'une quelconque des revendications 1 à 7, la source de lumière (715') ayant une extrémité proximale sur laquelle est disposé un élément de liaison (705') avec une saillie (710') s'étendant radialement vers l'extérieur à partir de celui-ci ;
l'appareil comprenant en outre un cathéter de distribution (750') avec une fente en spirale (760') définie longitudinalement à travers celui-ci ;
l'élément de liaison ayant un diamètre externe supérieur à un diamètre interne du cathéter de distribution ;
la saillie pouvant être reçue à l'intérieur de la fente provoquant la séparation de la fente, ce qui permet d'agrandir le diamètre interne du cathéter de distribution permettant à l'élément de liaison de rester en position à l'intérieur du corps lorsque le cathéter de distribution est retiré par traction dans une direction proximale.
